# EUROPEAN PATENT APPLICATION

(11) **EP 3 391 935 A1**
(43) Date of publication of application: **24.10.2018**
(21) Application number: 16875132.9
(22) Date of filing: 16.12.2016
(51) Int. Cl.: A61M 37/00

(54) **MICRONEEDLE AND MICRONEEDLE PATCH**

(30) Priority: 18.12.2015 JP 2015247640
(71) Applicant: Labo Juversa Co., Ltd., Chuo-ku Sapporo-shi Hokkaido 060-0001 (JP)
(72) Inventor: ONO, Ichiro, Sapporo-shi Hokkaido 060-0001 (JP)
(74) Representative: Hamer, Christopher K.
(86) International application number: PCT/JP2016/005166
(87) International publication number: WO 2017/104144

(57) **Abstract**

An object of the present invention is to provide a microneedle and a method for producing the same that are specialized for individual treatment purposes and can efficiently achieve a treatment effect. The microneedle according to the present invention relates to a microneedle including a drug containing layer, an intermediate layer, and a base layer in order from a tip side, wherein a boundary between the drug containing layer and the intermediate layer has a shape protruding toward the intermediate layer, and a thickness of a central portion of the intermediate layer is smaller than a thickness of a peripheral edge portion. The microneedle according to the present invention can be more easily broken in the intermediate layer than the conventional microneedle, thereby allowing the drug containing layer to be placed to properly target a particular area in epidermis or dermis. Also, drug release from the drug containing layer can be accurately controlled by a shape, a coating, or the like of the drug containing layer, and a range of application of treatment using a microneedle patch can be extended.

## Description

### Technical Field

The present invention relates to a microneedle for percutaneously delivering a predetermined drug into dermis, and a microneedle patch, with improved targeting of drug administration and improved administration efficiency.

### Background Art

In recent years, microneedles have been increasingly used in medical, aesthetic and health, and regenerative medicine fields. Specifically, a microneedle patch having a certain size and including a plurality of microneedles has been used to administer a target drug, vaccine, or the like, through, for example, a human body surface such as skin or mucosa into epidermis or dermis. Known methods for fabricating such microneedles include, for example, a method for filling a female mold having a plurality of recesses with a needle forming material using a squeegee and then drying and curing the material for fabricating microneedles (Patent Document 1), and an inkjet method for discharging droplets of a microneedle forming material into a female mold having a plurality of recesses by computer control using jet portions for an inkjet printer and then drying and curing the material for fabricating microneedles (Patent Document 2).

Purposes of drug administration using a microneedle include (1) targeting an inside of dermis per se, (2) administering a drug to an area rich in blood flow of a capillary network in papillary dermis, and absorbing the drug from capillaries of the area to administer the drug to blood vessels throughout the body, and (3) administering a drug to a boundary between epidermis and dermis in expectation of immunological action. However, although a microneedle is used for such various purposes, a specific method for fabricating a microneedle that is actually specialized for respective fields, efficiently administers a drug to a target area, and achieves a treatment effect has not yet been established.

### Prior Art Documents

### Patent Documents

Patent Document 1: Japanese unexamined Patent Application Publication No. 2012-200572
Patent Document 2: Japanese unexamined Patent Application Publication No. 2015-136422

### Summary of the Invention

### Object to be Solved by the Invention

An object of the present invention is to provide a microneedle and a microneedle patch that are specialized for individual treatment purposes and can efficiently achieve a treatment effect.

### Means to Solve the Object

The present inventors have diligently studied to solve the object, and found that a microneedle including a drug containing layer, an intermediate layer, and a base layer in order from a tip side, wherein a boundary between the drug containing layer and the intermediate layer has a shape protruding toward the intermediate layer can efficiently release a drug limitedly to a target area into dermis of skin and can accurately control time, and completed the present invention.

Specifically, the present invention is as described below.
(1) A microneedle including a drug containing layer, an intermediate layer, and a base layer in order from a tip side, wherein a boundary between the drug containing layer and the intermediate layer has a shape protruding toward the intermediate layer.
(2) The microneedle according to (1), wherein a thickness of a central portion of the intermediate layer is smaller than a thickness of a peripheral edge portion.
(3) The microneedle according to (1) or (2), wherein the boundary between the drug containing layer and the intermediate layer has a dome shape protruding toward the intermediate layer.
(4) The microneedle according to any one of (1) to (3), wherein the intermediate layer has a concave lens shape.
(5) The microneedle according to any one of (1) to (3), wherein the intermediate layer has an annular shape.
(6) The microneedle according to any one of (1) to (5), wherein a breaking strength of the intermediate layer is lower than a breaking strength of the drug containing layer and a breaking strength of the base layer.
(7) The microneedle according to (6), wherein the breaking strength of the intermediate layer is 0.9 times or less the breaking strength of the drug containing layer and the breaking strength of the base layer.
(8) The microneedle according to any one of (1) to (7), wherein a boundary between the intermediate layer and the base layer has a flat shape or a shape protruding toward the intermediate layer.
(9) The microneedle according to any one of (1) to (7), wherein a boundary between the intermediate layer and the base layer has a shape protruding toward the base layer.
(10) The microneedle according to any one of (1) to (9), wherein shapes of each of the boundaries between the drug containing layer, the intermediate layer, and the base layer are controlled by viscosity and surface activity level of a microneedle forming material, and/or water repellency of a surface of a concave original mold.
(11) The microneedle according to any one of (1) to (10), wherein a tip side of the drug containing layer further includes a tip layer that contains no drug.
(12) The microneedle according to (11), wherein a boundary between the tip layer and the drug containing layer has a flat shape or a shape protruding toward the tip layer.
(13) The microneedle according to (11) or (12), wherein a shape of the boundary between the tip layer and the drug containing layer is controlled by viscosity and surface activity level of a microneedle forming material, and/or water repellency of a surface of a concave original mold.
(14) The microneedle according to any one of (1) to (13), wherein the intermediate layer is dissolved after the drug containing layer is dissolved in a body.
(15) The microneedle according to any one of (1) to (14), wherein the drug containing layer is coated with a refractory polymer forming material having lower solubility in the body than the drug containing layer.
(16) The microneedle according to any one of (1) to (15), including two or more drug containing layers.
(17) The microneedle according to (16), wherein adjacent drug containing layers are partitioned by a boundary layer that contains no drug.
(18) The microneedle according to any one of (1) to (17), wherein an outer surface is coated with a coating material.
(19) The microneedle according to any one of (1) to (18), further including a partition wall made of a refractory polymer forming material around the drug containing layer,
   wherein the partition wall prevents crush of the drug containing layer when the microneedle is implanted, ensures that the drug containing layer is placed in a target area in a focused manner, and releases a drug with a predetermined time delay.
(20) The microneedle according to any one of (1) to (19), wherein a dissolving time of the drug containing layer and a dissolving time of the partition wall made of the refractory polymer forming material are controlled to control a drug release start time and a drug release time to achieve a programed continuous slow release of the drug.
(21) The microneedle according to any one of (1) to (20), which is used for targeting administration of the drug.
(22) A microneedle patch including a plurality of the microneedles according to any one of (1) to (21) provided on a substrate.

### Effect of the Invention

According to the present invention, a microneedle that is placed to properly target a particular area in epidermis and dermis and can control and adjust a drug administration time, and a microneedle patch including the microneedle and capable of efficiently administering a drug can be provided, thereby extending an application range of treatment using the microneedle patch.

### Brief Description of Drawings

[Figure 1] Figure 1 shows surface tension and a contact angle.
[Figure 2] Figure 2 shows shapes of a boundary when a microneedle is fabricated.
[Figure 3] Figure 3 shows states, after implantation, of microneedles 5, 6, 7 for treating subpapillary dermis, microneedles 8, 9, 10 for treating papillary dermis, and a microneedle 11 for interface of epidermis and upper dermis, with a broken surface of an intermediate layer being shown in plane for convenience.
[Figure 4] Figure 4 shows a fabrication method (upper row) and a state of use (lower row) of the microneedle 5 for treating subpapillary dermis.
[Figure 5] Figure 5 shows a fabrication method (upper row) and a state of use (lower row) of the microneedle 6 for treating subpapillary dermis.
[Figure 6] Figure 6 shows a fabrication method (upper row) and a state of use (lower row) of the microneedle 9 for treating papillary dermis.
[Figure 7] Figure 7 shows a fabrication method of the microneedle 10 for treating papillary dermis of a drug continuous slow release type.
[Figure 8] Figure 8 shows a state of use of the microneedle 10 for treating papillary dermis of a drug continuous slow release type.
[Figure 9] Figure 9 shows a fabrication method (upper row) and a state of use (lower row) of the microneedle 11 for epidermis and upper dermis.
[Figure 10] Figure 10 shows modes of a base layer and a substrate when a microneedle patch is fabricated.
[Figure 11] Figure 11 shows a microneedle formed such that a drug containing layer and the base layer are in direct contact with each other at a central portion of the intermediate layer and the intermediate layer has an annular shape.
[Figure 12] Figure 12 shows broken surfaces of microneedles, after use, fabricated by varying a boundary shape in Example 1. A has a flat broken surface, B has a broken surface with a recessed central portion, and C has a broken surface with a protruding central portion.
[Figure 13] Figure 13 is an enlarged view of broken surfaces of microneedles, after use, fabricated by varying a boundary shape in Example 1. A has a flat broken surface, B has a broken surface with a recessed central portion, and C has a broken surface with a protruding central portion.
[Figure 14] Figure 14 is an enlarged view of broken surfaces of microneedles (A) for treating subpapillary dermis without an intermediate layer and microneedles (B) having an annular intermediate layer in Example 2. For A, residual lengths are different, while for B, the microneedles are broken to have uniform residual lengths, and an annular intermediate layer 32 partially remains at a peripheral edge of the broken surface.

### Mode of Carrying Out the Invention

The present invention relates to a microneedle for percutaneously administering a drug, including a drug containing layer, an intermediate layer, and a base layer in order from a tip side, wherein a boundary between the drug containing layer and the intermediate layer has a shape protruding toward the intermediate layer. The boundary between the drug containing layer and the intermediate layer is formed into a shape protruding toward the intermediate layer, preferably a dome shape, and further preferably a half-spindle shape, thereby causing stress to be easily applied in the intermediate layer when the microneedle is inserted into skin and the base layer is pulled obliquely. Thus, a microneedle patch including the microneedle according to the present invention provided on a substrate is applied to the skin, and the substrate is removed from the skin, thereby causing the microneedle to be easily broken in the intermediate layer and causing the drug containing layer to be reliably placed in a target area. A boundary between the intermediate layer and the base layer may have a flat shape or a shape protruding toward the intermediate layer or the base layer. It is preferable that a central portion of the intermediate layer is designed to be thinner than a peripheral edge portion because the microneedle has a strong structure against vertical stress when inserted into the skin, while when lateral stress is applied after implantation, the intermediate layer acts like a wedge and the microneedle is easily broken in the intermediate layer. More preferably, the intermediate layer is designed to have a concave lens shape. Also, the central portion of the intermediate layer may be an extremely thin layer, or the base layer may be in direct contact with the drug containing layer at the central portion of the intermediate layer. For the latter case, the intermediate layer has an annular shape with a thick outside, and an inner diameter thereof may be preferably 5 µm or more, more preferably 10 µm or more, and further preferably 50 µm or more.

In the microneedle according to the present invention, a tip side of the drug containing layer may further include a tip layer containing no drug for controlling a position and a shape of the drug containing layer and a release time of the drug or improving penetrability.

The microneedle according to the present invention may be produced using any known methods including, for example, a method for fabricating a concave original mold including a plurality of recesses having a shape of microneedles, and filling the concave original mold with a microneedle forming material using a dispenser, an inkjet device, a squeegee, or the like.

Any biodegradable polymer may be used as a material for forming the tip layer, the drug containing layer, and the intermediate layer of the microneedle according to the present invention as long as it is degraded in body after implanted into the skin to release a drug. The biodegradable polymer may include, for example, protein such as gluten or gelatin, polysaccharides such as hyaluronic acid, chondroitin sulfate, alginic acid, starch, or dextran, polylactic acid, polyglycolic acid, lactic acid-glycolic acid copolymer, polycaprolactone, polyhydroxybutyrate, polyethylene glycol, or polypropylene glycol, and may be one polymerized with a drug to control solubility of biodegradable polymer, thereby allowing continuous slow release of the drug. The base layer may be formed of the biodegradable forming material listed above, or a non-biodegradable forming material.

In the microneedle according to the present invention, breaking strength of the intermediate layer is preferably lower than breaking strength of the drug containing layer and the base layer, and may be, for example, 0.9 times or less, 0.8 times or less, 0.7 times or less, 0.6 times or less, or 0.5 times or less the breaking strength of the drug containing layer and the base layer. The intermediate layer is preferably formed of a material that is less soluble in the body than the drug containing layer. The intermediate layer formed of such a material serves as a partition wall that prevents the drug from spreading to areas other than the target area in the body. A thickness of the intermediate layer is preferably 50 to 200 µm, more preferably 80 to 160 µm, and further preferably 100 to 150 µm at a peripheral edge portion of the microneedle, and preferably 50 µm, less than 50 µm, more preferably 30 µm or less, and more preferably 10 µm or less, and may be 0 µm at the central portion of the microneedle.

Drugs contained in the drug containing layer may include, but not limited to, for example, antidiabetic drugs, antihypertensive agents, anticancer agent, antiallergic agents, hormones, growth factors, vaccines, analgesics such as morphine, anti-inflammatory analgesics, drugs for treatment of osteoporosis, drugs for treatment of anemia, drugs for treatment of hypercholesterolemia, biological products, gene therapy agents, drugs for emergency treatment such as adrenaline, and allergen testing materials. In particular, drugs that require subdermal injection can be favorably exemplified since the microneedle can be used to administer a drug into dermis through an insertion hole formed in the skin. The content of the drug may be determined according to its type or form and in view of continuous slow release or the like.

A shape of the drug containing layer and an area from a tip part may be freely controlled by changing a boundary between the tip layer and the drug containing layer, a boundary between the drug containing layer and the intermediate layer, lengths of the tip layer and the drug containing layer, or the like, and may include, but not limited to, a rod shape, a triangular pyramidal shape, an inverted truncated conical shape, an inverted truncated pyramidal shape, a spindle shape, an umbrella shape, a dumbbell shape, a spherical shape, and a circular flat plate shape. A longer drug containing layer provides a larger side surface area, and thus allows quick dissolving to release a drug and allows broad drug administration in a vertical direction over dermal layer. On the other hand, a vertically shorter drug containing layer provides a smaller side surface area, which is advantageous for continuous slow release of the drug and allows drug administration limitedly to a narrow area such as papillary dermis.

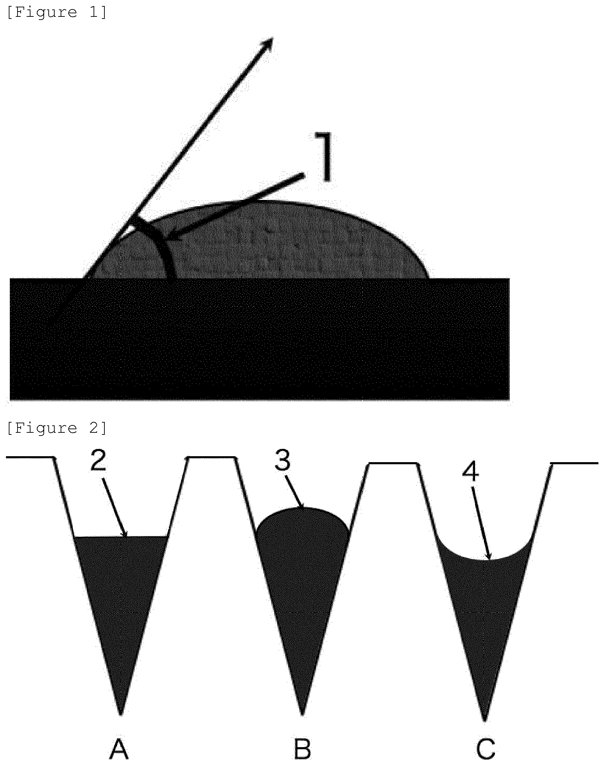

In the case where forming materials are sequentially overlaid in the concave original mold to fabricate a microneedle, for example, the shapes of the boundaries between the tip layer, the drug containing layer, the intermediate layer, and the base layer are determined according to a contact angle 1 between a forming material previously injected into the concave original mold and the concave original mold (Figure 1). The contact angle 1 depends on viscosity or surface activity of the forming material and/or water repellency of a surface of the concave original mold. Figure 2 shows a flat boundary state 2, a protruding state 3, and a recessed state 4. If the forming material previously injected into the concave original mold has high surface activity, the boundary protrudes toward the tip side, and if the forming material previously injected into the concave original mold has low surface activity, the boundary protrudes toward the base layer. With higher viscosity of the forming material previously injected into the concave original mold, the boundary protrudes more toward the base layer. The shape of the boundary can be controlled by a material of the concave original mold. For a material for the concave original mold having higher water repellency, the boundary tends to protrude toward the base layer with a larger contact angle 1, while for a material having lower water repellency, the boundary is recessed more so that the contact angle 1 is 0 or negative. The concave original mold may be made of any materials, and silicon, plastic, or metal may be favorably exemplified. A surface of the concave original mold may be subjected to water repellent finishing or hydrophilization as required. In the case of fabricating the microneedle using an inkjet method, controlling a dropping amount, a dropping area, and an interval of dropping in addition to the viscosity or the surface activity of the forming material can control the shape of the boundary more accurately. Also, controlling an interval of drying and curing according to an intended use of the microneedle allows fabrication into an optimum shape for the intended use.

The surface activity of the microneedle forming material can be controlled by adding surfactant to the forming material. The surfactants usable in the present invention may be surfactants that can be added to medicinal products, and may include non-ionic, anionic, cationic, and amphoteric surfactants.

The viscosity of the microneedle forming material may be controlled by adding a thickener or a viscosity reducer to the forming material. The thickener usable in the present invention may be a thickener that can be added to medicinal products, and may include monosaccharides such as sucrose or dextrose, disaccharide and oligosaccharide, pectin, carrageenan, guar gum, locust bean gum, tamarind gum, xanthan gum, curdlan, polysaccharides such as chondroitin sulfate, alginic acid, starch, or dextran, and protein such as gluten or gelatin. The viscosity reducer usable in the present invention may be a viscosity reducer that can be added to medicinal products, and may include urea, lower alcohol such as ethanol, and emulsifier. Reducing concentration of the forming material can reduce the viscosity.

The drug containing layer may be coated with a refractory polymer forming material having lower solubility in the body than the drug containing layer to control a drug release time in the body. The drug containing layer may be coated by forming the tip layer and the intermediate layer adjacent to the drug containing layer of the refractory polymer forming material, coating around the drug containing layer with the refractory polymer forming material after fabrication of the microneedle, and so on. Also, in the case where the forming materials are sequentially overlaid in the concave original mold to fabricate a microneedle, a tip layer forming material is injected and dried, then the refractory polymer forming material is sprayed or applied on an upper portion of the tip layer and a wall surface of the concave original mold and dried, then a drug containing layer forming material is injected into a central portion of the concave original mold and dried, and the refractory polymer forming material is further overlaid, thereby allowing the drug containing layer to be coated with the refractory polymer forming material. In this case, increasing surface activity of the refractory polymer forming material increases affinity between the refractory polymer forming material and the concave original mold, thereby facilitating coating of the drug containing layer.

One drug containing layer or two or more drug containing layers may be included. For the two or more drug containing layers, the drug containing layers may contain the same drug or two or more types of different drugs, and adjacent drug containing layers may be partitioned by a boundary layer containing no drug. The two or more drug containing layers are formed of materials having different solubility in the body, thereby allowing the same or different drugs to be released with a time lag. Thus, a microneedle can be fabricated capable of a time-lag medical treatment using two or more types of drugs contained.

In the microneedle according to the present invention, hardness of the tip layer is preferably higher than hardness of the drug containing layer, and may be, for example, 1.1 times or more, 1.2 times or more, 1.3 times or more, 1.4 times or more, 1.5 times or more the hardness of the drug containing layer. Increasing the hardness of the tip layer improves penetrability of the microneedle, and allows the drug containing layer to more reliably reach the target area. A material for forming the tip layer may be the same as or different from a material for forming the drug containing layer except a drug. For the same material, the hardness of the tip layer containing no drug is higher than hardness of the drug containing layer containing a drug. The hardness of the tip layer may be increased by adding a curing agent to the forming material.

In the present invention, to improve penetrability of the microneedle, dryness of only the tip layer may be increased by increasing a drying and curing time. Increasing the dryness of only the tip layer can increase the hardness and reduce a radius of curvature of the tip. The tip may be drawn and extended to reduce the radius of curvature. The radius of curvature of the tip of the microneedle according to the present invention is preferably 10 µm or less, more preferably 7 µm or less, further preferably 5 µm or less, and particularly preferably 3 µm or less. The section of the tip layer may be formed into, not limited to a circular shape, an elliptical shape, a triangular shape, a square shape, a plus shape, an asterisk shape, or the like. The tip may be obliquely cut to increase sharpness. A helical groove may be continuously formed in an outside of the microneedle so that the microneedle is rotated by implantation, thereby facilitating the implantation of the microneedle, and promoting breakage of the microneedle in the intermediate layer.

To smooth and cure a surface and improve penetrability, an outer surface of the microneedle according to the present invention may be coated with a coating material selected from biodegradable polymer selected from protein such as gluten or gelatin, polysaccharides such as chondroitin sulfate, alginic acid, starch, or dextran, polylactic acid, polyglycolic acid, lactic acid-glycolic acid copolymer, polycaprolactone, polyhydroxybutyrate, polyethylene glycol, and polypropylene glycol, silicon compound, surfactants selected from amphoteric, anionic, cationic, and non-ionic surfactants, ethyl alcohol, saline, and water. Coating with the coating material may be performed by any known methods including a dip coating method, an inkjet method, and a spray coating method. A thickness of the coating may be, for example, 10 µm or less, preferably 5 µm or less, and more preferably 3 µm or less. Friction resistance is preferably lower by 5% or more, more preferably 10% or more, and further preferably 20% or more than that before coating with the coating material. The coating material may contain a curing agent or a target drug. A coating material having lower solubility in the body than the drug containing layer may be used for continuous slow release of the drug. The coating material is desirably a material that can improve penetrability of the microneedle and maintain sharpness of the microneedle during a storage period from after production to use.

The curing agent in the present invention refers to a material that can increase the hardness of the microneedle. The curing agent may include, for example, calcium chloride, sodium chloride, polysaccharides, hyaluronic acid, chondroitin sulfate, carboxy polymer, polyacrylic acid, polylactic acid, hydroxyapatite, polyethylene glycol, fluorine compound, and silicon compound. The curing agent may be added to the microneedle forming material or to the coating material, and an amount of use of the curing agent may be set appropriately according to a type and intended use thereof. When the curing agent is a solid material, the material having a particle size of 5 µm or less, preferably 3 µm or less, and more preferably 1 µm or less is preferably used to smooth a surface of the microneedle. The curing agent is desirably a material that can improve shape retainability of the microneedle and maintain sharpness of the microneedle during a storage period from after production to use.

A total length of the microneedle according to the present invention may be determined according to purposes of drug administration. For example, in the case of drug administration targeting an inside of dermis per se, the drug is desirably administered to reticular dermis at a deep area of dermis, and the total length of the microneedle is preferably 400 to 1000 µm, more preferably 450 to 800 µm, and further preferably 500 to 650 µm. In the case of administering a drug to an area rich in blood flow of a capillary network in papillary dermis, and absorbing the drug from capillaries of the area to administer the drug to blood vessels throughout the body in expectation of an effect, the total length of the microneedle is preferably 100 to 600 µm, more preferably 150 to 500 µm, and further preferably 200 to 450 µm such that the drug can be administered to papillary dermis and subpapillary dermis. In the case of administering a drug to a boundary between epidermis and dermis in expectation of immunological action, the total length of the microneedle is preferably 50 to 300 µm, more preferably 100 to 250 µm, and further preferably 150 to 200 µm such that the drug can be administered to the interface between epidermis and dermis. A diameter, a long diameter, and a length of a long side of the base part are preferably 30 to 1000 µm, more preferably 150 to 500 µm, and further preferably 200 to 350 µm.

In the present invention, a microneedle patch refers to a patch including a plurality of the microneedles provided on a substrate. The substrate can be exemplified by a sheet consisting of biodegradable polymer selected from protein such as gluten or gelatin, polysaccharides such as chondroitin sulfate, alginic acid, starch, or dextran, polylactic acid, polyglycolic acid, lactic acid-glycolic acid copolymer, polycaprolactone, polyhydroxybutyrate, polyethylene glycol, and polypropylene glycol, or non-biodegradable polymer selected from polyolefin such as polyethylene or polypropylene, polystyrene, polyester, polyurethane, polyamide, and fluorocarbon resin, paper, nonwoven fabric, or cloth. The microneedle patch according to the present invention may have an adhesive surface, and may have a structure including the substrate and the base layer integrated with each other. Such a structure ensures breakage of the microneedle in the intermediate layer.

Now, an embodiment of the present invention will be described with reference to the drawings.

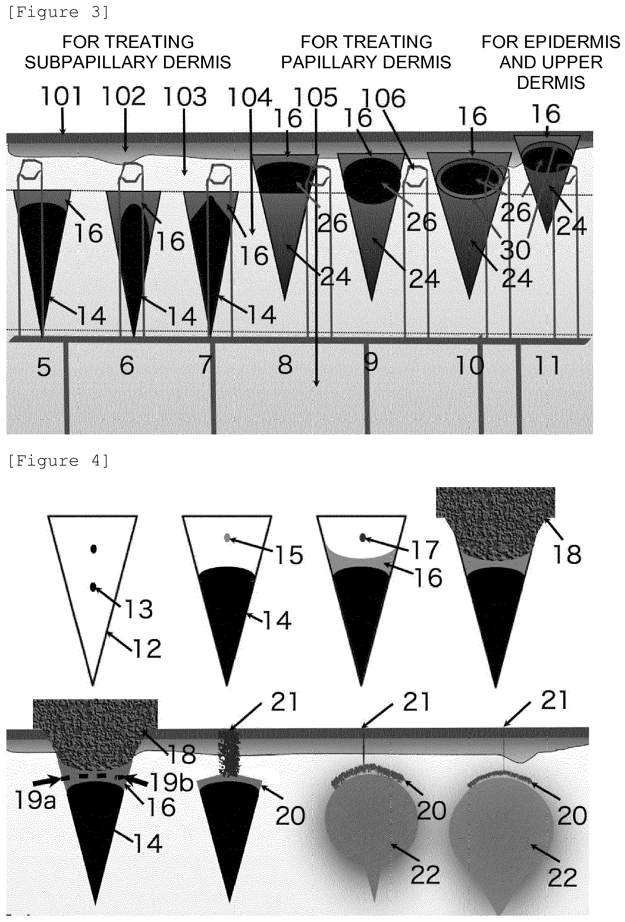

Figure 3 shows states of microneedles for different purposes of treatment after implantation into dermis. Microneedles 5, 6, 7 are for treating subpapillary dermis 104, microneedles 8, 9, 10 are for absorbing a drug from a capillary network 106 of papillary dermis 103, and a microneedle 11 is for administering a drug to an interface between epidermis and dermis. The microneedles 5, 6, 7 are implanted such that a drug containing layer 14 reaches deep into the dermis beyond the papillary dermis. At this time, since the drug can be prevented from being absorbed from the capillary network 106 in the papillary dermis 103, the drug containing layer 14 stays long in the dermis, thereby easily providing a predetermined effect. On the other hand, a drug containing layer 14 of the microneedles 8, 9, 10 can accurately reach the papillary dermis 103 to administer a drug throughout the body via the capillary network 106 in the papillary dermis 103. The microneedle 11 can limitedly administer a drug to around an interface between the epidermis 102 and the papillary dermis 103 to cause immune response or for an allergen test.

A method for fabricating a microneedle using an inkjet method will be mainly descried below. However, even when other methods such as a squeegee method are used, a microneedle having a boundary with a controlled shape can be similarly fabricated by performing a step of injecting a microneedle forming material into a microneedle fabricating concave original mold with controlled water repellency and a step of drying like the inkjet method. Figure 4 shows, in an upper row, a method for fabricating the microneedle 5 for treating subpapillary dermis using the inkjet method. A drug containing layer forming material 13 having high viscosity and containing a drug is repeatedly discharged into a microneedle fabricating concave original mold 12 to fabricate a drug containing layer 14 with a protruding boundary. A tip part is dried and cured, and then an intermediate layer forming material 15 having low surface tension and having surface activity is discharged to form an intermediate layer 16 with a recessed boundary. After drying and curing, a base layer forming material 17 is further discharged and integrated with a substrate to fabricate a base layer 18.

Figure 4 shows, in a lower row, treatment using the microneedle 5. When a microneedle patch including the microneedle 5 is implanted into the skin using an impact applicator or the like, broken surfaces 19a, 19b are formed in the intermediate layer 16. Removing the microneedle patch allows only the drug containing layer 14 and a part 20 of the intermediate layer to be placed in subpapillary dermis. Then, the drug is released from the drug containing layer 14 as shown by 22. In this case, until an insertion hole 21 closes, the part 20 of the intermediate layer exists on an epidermis side of the drug containing layer 14, and dissolves later than the drug containing layer 14, thereby preventing spreading of the drug to papillary dermis rich in blood vessels or leakage from the insertion hole 21.

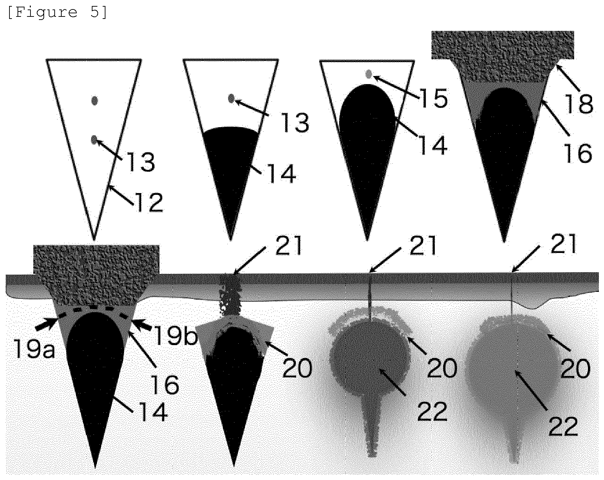

Figure 5 shows, in an upper row, a method for fabricating the microneedle 6 for treating subpapillary dermis using the inkjet method. A drug containing layer forming material 13 having high viscosity and containing a drug is repeatedly discharged into a microneedle fabricating concave original mold 12 to fabricate a drug containing layer 14 with a protruding boundary. After drying, the drug containing layer forming material 13 is continuously repeatedly discharged to a center of the boundary such that the boundary significantly protrudes, and an intermediate layer side of the drug containing layer is formed into a half-spindle shape. The drug containing layer 14 is dried and cured, and then an intermediate layer forming material 15 having low surface tension and having surface activity is discharged to form an intermediate layer 16 with a recessed boundary. After further drying and curing, a base layer forming material 17 is discharged and integrated with a substrate to fabricate a base layer 18.

Figure 5 shows, in a lower row, treatment using the microneedle 6. When a microneedle patch including the microneedle 6 is implanted into the skin using an impact applicator or the like, broken surfaces 19a, 19b are formed in the intermediate layer 16. Removing the microneedle patch allows only the drug containing layer 14 and a part 20 of the intermediate layer to be placed in subpapillary dermis. Then, the drug is released from the drug containing layer 14 as shown by 22. In this case, until an insertion hole 21 closes, the part 20 of the intermediate layer exists on an epidermis side of the drug containing layer 14, and dissolves later than the drug containing layer 14, thereby preventing spreading of the drug to papillary dermis rich in blood vessels or leakage from the insertion hole 21.

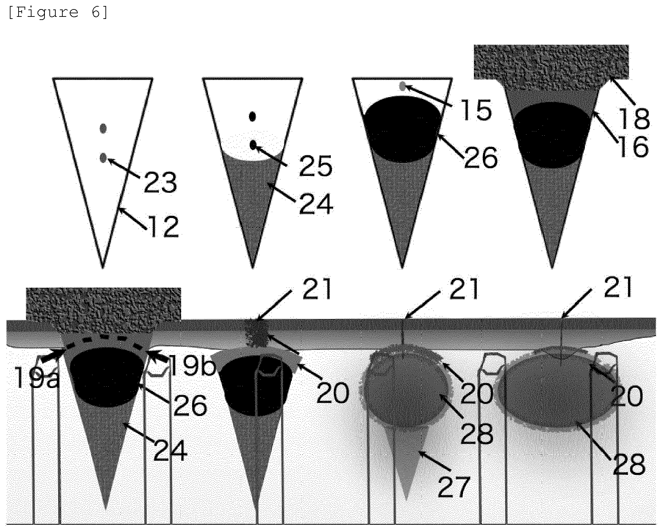

Figure 6 shows, in an upper row, a method for fabricating the microneedle 9 for treating papillary dermis using the inkjet method. A tip layer forming material 23 having low viscosity and high surface activity is repeatedly discharged into a microneedle fabricating concave original mold 12 to fabricate a tip layer 24 with a recessed boundary. After the boundary with a recessed center is dried and cured, a drug containing layer forming material 25 having high viscosity and containing a drug is discharged to form a drug containing layer 26 with a protruding boundary. After drying and curing, an intermediate layer forming material 15 having low surface tension and having surface activity is discharged to form an intermediate layer 16 with a recessed boundary. Further, a base layer forming material 17 is discharged and integrated with a substrate to fabricate a base layer 18. In this case, controlling solubility of the drug containing layer 26 can achieve continuous slow release.

Figure 6 shows, in a lower row, treatment using the microneedle 9. When a microneedle patch including the microneedle 9 is implanted into the skin using an impact applicator or the like, broken surfaces 19a, 19b are formed in the intermediate layer 16. Removing the microneedle patch allows the tip layer 24, the drug containing layer 26, and a part 20 of the intermediate layer to be placed in papillary dermis. Then, the tip layer 24 dissolves in an early stage as shown by 27, and the drug is released from the drug containing layer 26 as shown by 28. In this case, until an insertion hole 21 closes, the part 20 of the intermediate layer exists on an epidermis side of the drug containing layer 26, and dissolves later than the drug containing layer 26, thereby preventing leakage of the drug from the insertion hole 21. Thus, the administered drug is absorbed into blood vessels throughout the body to allow efficient treatment. In the upper row in Figure 6, the tip layer forming material 23 having low viscosity and high surface activity may be replaced by a tip layer forming material having moderate viscosity and having no surface activity to fabricate the microneedle 8 having a tip layer 24 with a flat boundary.

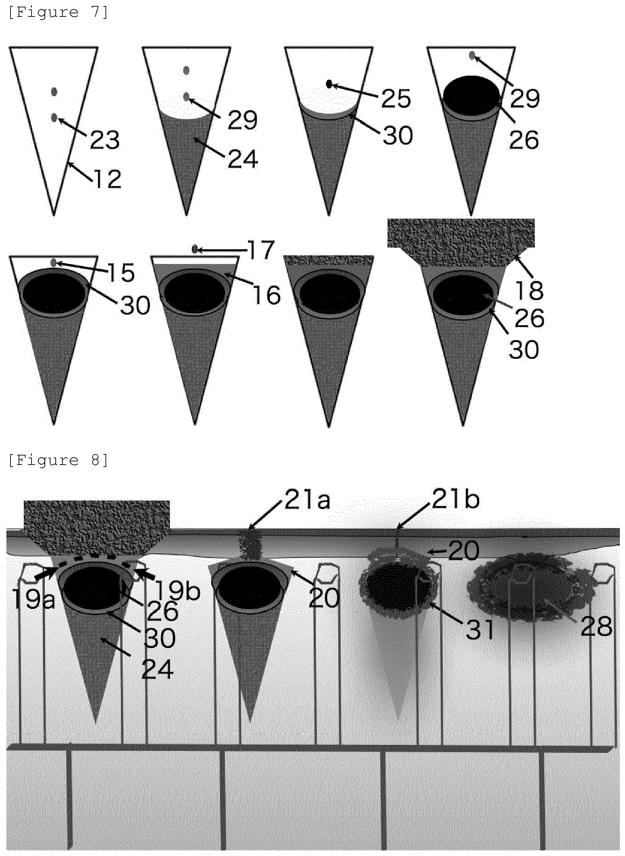

Figure 7 shows a method for fabricating the microneedle 10 for papillary dermis in which a drug containing layer 26 is coated with a partition wall 30 made of a refractory polymer forming material, using the inkjet method. A tip layer forming material 23 having low viscosity and high surface activity is repeatedly discharged into a microneedle fabricating concave original mold 12 to fabricate a tip part 24 with a recessed boundary. After the boundary with a recessed center is dried and cured, a refractory polymer forming material 29 having moderate or high surface activity and that slowly dissolves in the body is discharged to fabricate a lower half of the partition wall 30 made of the refractory polymer forming material. Further, a drug containing layer forming material 25 having high viscosity and containing a drug is discharged to form a drug containing layer 26 with a protruding boundary. Then, the refractory polymer forming material 29 having moderate or high surface activity and that slowly dissolves in the body is discharged to fabricate an upper half of the partition wall 30 made of the refractory polymer forming material. After drying and curing, an intermediate layer forming material 15 having low surface tension and having surface activity is discharged to form an intermediate layer 16 with a recessed boundary. Further, a base layer forming material 17 is discharged and integrated with a substrate to fabricate a base layer 18.

Figure 8 shows treatment using the microneedle 10. When a microneedle patch including the microneedle 10 is implanted into the skin using an impact applicator or the like, broken surfaces 19a, 19b are formed in the intermediate layer 16. Removing the microneedle patch allows the tip layer 24, the drug containing layer 26 coated with the partition wall 30 made of the refractory polymer forming material, and a part 20 of the intermediate layer to be placed in papillary dermis. Controlling solubility of the drug containing layer 26 and controlling solubility of the partition wall 30 made of the refractory polymer forming material can control both a drug release start time and a drug release time from the drug containing layer 26, thereby allowing complete control of drug administration. In this case, the drug containing layer 26 is coated with the partition wall 30 to prevent crush of the drug containing layer 26 in implantation and ensure that the drug containing layer 26 is placed at a target depth in the skin within a more limited range. Also, independently controlling a dissolving time of the drug containing layer 26 and a dissolving time of the partition wall 30 made of the refractory polymer forming material can control a drug release start time and a drug release time, thereby achieving programmed continuous slow release of the drug. Then, the drug is released from the drug containing layer 26 as shown by 28. The partition wall 30 made of the refractory polymer forming material can control the drug release time from the drug containing layer 26, thereby allowing continuous slow release of the drug and delayed drug administration. In this case, until an insertion hole 21 closes, the part 20 of the intermediate layer exists on an epidermis side of the drug containing layer 26, and dissolves later than the drug containing layer 14, thereby preventing leakage of the drug from the insertion hole 21. Thus, the administered drug is absorbed into blood vessels throughout the body to allow efficient treatment.

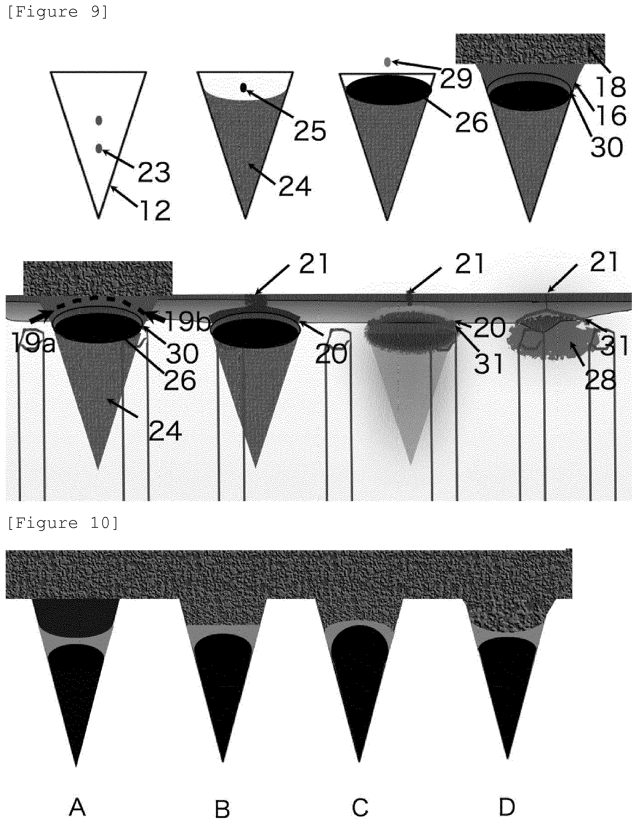

Figure 9 shows, in an upper row, a method for fabricating the microneedle 11 for interface of epidermis and upper dermis using the inkjet method. A tip layer forming material 23 having low viscosity and high surface activity is repeatedly discharged into a microneedle fabricating concave original mold 12 to fabricate a tip layer 24 with a recessed boundary. After the boundary with a recessed center is dried and cured, a drug containing layer forming material 25 having high viscosity and containing a drug is discharged to form a drug containing layer 26 with a protruding boundary. Then, a refractory polymer forming material 29 having high surface activity and that slowly dissolves in the body is discharged to fabricate a partition wall 30 made of the refractory polymer forming material. Then, an intermediate layer forming material 25 having low surface tension and having surface activity is discharged to form an intermediate layer 16 with a recessed boundary. Further, a base layer forming material is discharged and integrated with a substrate to fabricate a base layer 18. In this case, an operation for fabricating the partition wall 30 may be omitted depending on types of drugs contained or purposes of administration.

Figure 9 shows, in a lower row, treatment using the microneedle 11. When a microneedle patch including the microneedle 11 is implanted into the skin using an impact applicator or the like, broken surfaces 19a, 19b are formed in the intermediate layer 16. Removing the microneedle patch allows the tip part 24, the drug containing layer 26, the partition wall 30 made of the refractory polymer forming material, and a part 20 of the intermediate layer to be placed in papillary dermis. Then, the drug is released from the drug containing layer 26. Controlling solubility of the drug containing layer 26 and controlling solubility of the partition wall 30 made of the refractory polymer forming material can control both a drug release start time and a drug release time from the drug containing layer 26, thereby allowing complete control of drug administration. In this case, the drug containing layer 26 is coated with the partition wall 30 to prevent crush of the drug containing layer 26 in administration and ensure that the drug containing layer 26 is placed at a target depth in the skin within a more limited range. Also, independently controlling a dissolving time of the drug containing layer 26 and a dissolving time of the partition wall 30 made of the refractory polymer forming material can control a drug release start time and a drug release time, thereby achieving programmed continuous slow release of the drug. In this case, until an insertion hole 21 closes, the part 20 of the intermediate layer exists on an epidermis side of the drug containing layer 26, and dissolves later than the drug containing layer 26, thereby preventing leakage of the drug from the insertion hole 21.

Figure 10 shows a method for forming a base layer and a substrate. When a microneedle array is fabricated by the inkjet method, the base layer and the substrate may be separately or integrally fabricated. As shown in A, when separately fabricated, the base layer and the substrate can be easily fabricated. However, the microneedle disadvantageously tends to be peeled at a bonded portion between the base layer and the substrate and destroyed. On the other hand, integrally fabricating the base layer and the substrate as shown in D can overcome such a disadvantage. In both fabrication methods, a boundary between an intermediate layer and the base layer may have a shape of a flat surface as shown in B, a recessed surface as shown in C, or a protruding surface as shown in A and D.

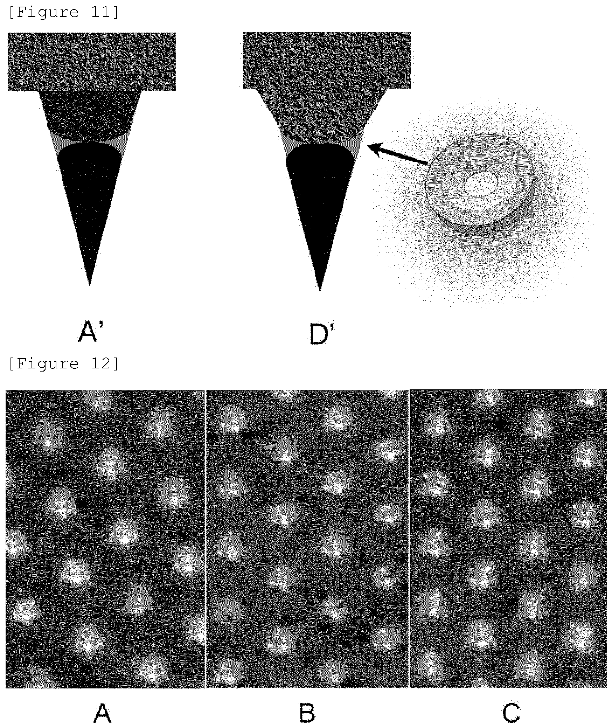

Figure 11 shows a mode in which the drug containing layer and the base layer are in direct contact with each other at a central portion of the intermediate layer. A' shows a mode in which the base layer and the substrate are separately fabricated, and D' shows a mode in which the base layer and the substrate are integrally fabricated. In these modes, the intermediate layer has an annular shape as shown in a perspective view on the right in Figure 11.

Now, the present invention will be described more specifically with reference to examples, but a technical scope of the present invention is not limited to the examples.

### Example 1

In Example 1, methods for fabricating a flat boundary, a boundary with a drug containing layer protruding toward a base layer, and a boundary with a recessed drug containing layer in a two-layer microneedle consisting of a drug containing layer and a base layer were studied. In this example, the microneedle was fabricated by the inkjet method using a mixed material of dextran and chondroitin sulfate as a microneedle forming material, and a combination of a mixed material of dextran and chondroitin sulfate and a mixture of basic fibroblast growth factor (bFGF) and sucrose as a drug containing layer.

A drug containing layer forming material (a mixture of dextran and chondroitin sulfate in a ratio of 2:2) prepared to have moderate viscosity and have no surface activity was discharged into a microneedle fabricating concave original mold 12 using inkjet to fabricate a drug containing layer with a flat boundary, and after drying, a base layer forming material was overlaid on the drug containing layer to fabricate a microneedle A including the drug containing layer with the flat boundary. A drug containing layer forming material (a mixture of dextran and chondroitin sulfate in a ratio of 3:1) prepared to have high viscosity was discharged into a microneedle fabricating concave original mold 12 using inkjet to fabricate a drug containing layer with a protruding boundary, and after drying, a base layer forming material was overlaid on the drug containing layer to fabricate a microneedle B including the drug containing layer with the protruding boundary. A drug containing layer forming material (a mixture of dextran and chondroitin sulfate and also polyethylene glycol 600 in a ratio of 2:1:1) prepared to have low viscosity and high surface activity was discharged into a microneedle fabricating concave original mold 12 using inkjet to fabricate a drug containing layer with a recessed boundary, and after drying, a base layer forming material was overlaid on the drug containing layer to fabricate a microneedle C including the drug containing layer with the recessed boundary. In this example, the content of the drug was set to 1.2 µg per 1 patch.

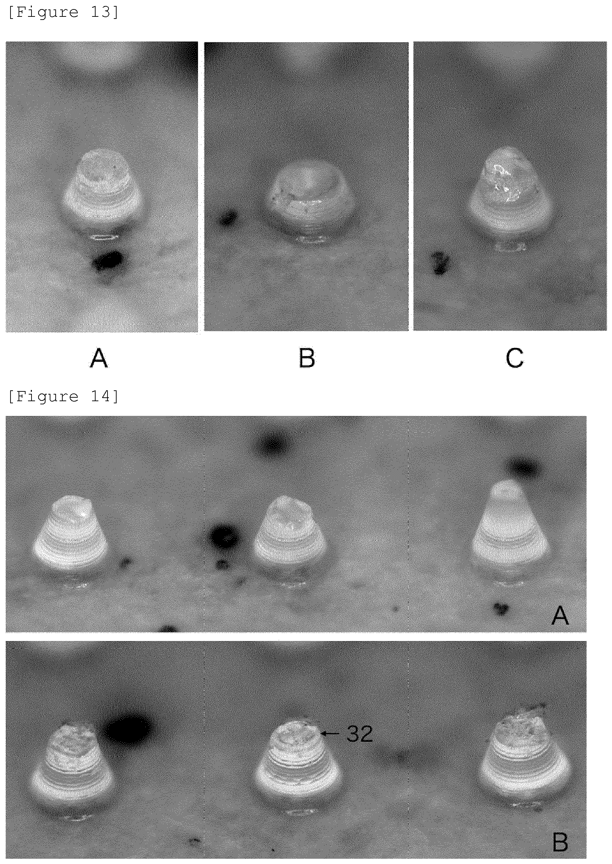

Figures 12 and 13 show broken surfaces of the microneedles A, B, C after use. A had a flat broken surface, B had a broken surface with a recessed central portion, and C had a broken surface with a protruding central portion. This example demonstrated that controlling water repellency of the microneedle fabricating concave original mold 12 and viscosity and surface activity of the forming material can control the shape of the boundary.

### Example 2

In Example 2, states of broken surfaces, after use, of a microneedle (A) without an intermediate layer, and a microneedle (B) formed such that a drug containing layer and a base layer are in direct contact with each other at a central portion of the intermediate layer, and the intermediate layer has an annular shape as shown in Figure 11 were compared.

The microneedle (A) without an intermediate layer was fabricated, similarly to Example 1, in such a manner that a drug containing layer forming material (a mixture of dextran and chondroitin sulfate in a ratio of 2:2) prepared to have moderate viscosity and have no surface activity was discharged into a microneedle fabricating concave original mold 12 using inkjet to fabricate a drug containing layer with a flat boundary, and after drying, a base layer forming material was overlaid on the drug containing layer.

The microneedle (B) formed such that the drug containing layer and the base layer are in direct contact with each other at the central portion of the intermediate layer, and the intermediate layer has an annular shape was fabricated in such a manner that droplets of a drug containing layer forming material (a mixture of dextran and chondroitin sulfate in a ratio of 3:1) containing a drug and having high viscosity and high surface activity was sprayed into a microneedle fabricating concave original mold 12 to fabricate a drug containing layer with a protruding central portion. After drying, a small amount of intermediate layer forming material (a mixture of dextran and chondroitin sulfate and also polyethylene glycol 600 in a ratio of 2:1:1) having low viscosity and low surface activity was sprayed to fabricate an annular intermediate layer. Immediately before sufficient drying, a base layer forming material having high viscosity was overlaid to fabricate a microneedle of a three-layer structure including the annular intermediate layer. These microneedles were all fabricated by the inkjet method using a mixed material of dextran and chondroitin sulfate. In this example, the content of the drug was set to 1.8 µg per 1 patch.

Figure 14 shows enlarged photographs of a microneedle patch including the microneedle (A) without an intermediate layer, and a microneedle patch including the microneedle (B) having the annular intermediate layer after implantation. For A, adjacent microneedles had different broken areas and no uniformity was found, while for B, extremely high uniformity of position and shape of broken surfaces was found, and an annular intermediate layer 32 remaining at a peripheral edge of the broken surface was able to be observed.

### Industrial Applicability

The microneedle according to the present invention can be more easily broken in the intermediate layer than the conventional microneedle, thereby allowing the drug containing layer to be placed to properly target a particular area in epidermis or dermis. Any known methods for fabricating a microneedle can be applied. Besides, a drug containing position in the drug containing layer and drug release can be accurately controlled by the shape, the coating, or the like of the drug containing layer, and a range of application of treatment using the microneedle patch can be extended. Thus, the microneedle according to the present invention has high industrial usability in a medical field.

### Explanation of Letters or Numerals

1 contact angle
2 flat boundary
3 protruding boundary
4 recessed boundary
5, 6, 7 state of microneedle for subpapillary dermis after implantation
8, 9, 10 state of microneedle for papillary dermis after implantation
11 state of microneedle for epidermis and upper dermis after implantation
12 microneedle fabricating concave original mold
13 droplet of drug containing layer forming material
14 drug containing layer
15 droplet of intermediate layer forming material
16 intermediate layer
17 droplet of base layer forming material
18 base layer fabricated integrally with substrate
19 broken surface
20 part of intermediate layer after breakage
21 insertion hole
22 drug containing layer dissolved to release drug
23 droplet of tip layer forming material
24 tip layer
25 droplet of drug containing layer forming material
26 drug containing layer
27 dissolved tip layer
28 drug containing layer dissolved to release drug
29 droplet of refractory polymer forming material
30 partition wall made of refractory polymer forming material
31 partition wall made of refractory polymer forming material (during being dissolved)
32 annular intermediate layer remaining at peripheral edge of broken surface after administration
101 horny cell layer
102 epidermis
103 papillary dermis
104 subpapillary dermis
105 reticular dermis
106 capillary network of papillary dermis

## Claims

1. A microneedle comprising a drug containing layer, an intermediate layer, and a base layer in order from a tip side,
wherein a boundary between the drug containing layer and the intermediate layer has a shape protruding toward the intermediate layer.

2. The microneedle according to claim 1, wherein a thickness of a central portion of the intermediate layer is smaller than a thickness of a peripheral edge portion.

3. The microneedle according to claim 1 or 2, wherein the boundary between the drug containing layer and the intermediate layer has a dome shape protruding toward the intermediate layer.

4. The microneedle according to any one of claims 1 to 3, wherein the intermediate layer has a concave lens shape.

5. The microneedle according to any one of claims 1 to 3, wherein the intermediate layer has an annular shape.

6. The microneedle according to any one of claims 1 to 5, wherein a breaking strength of the intermediate layer is lower than a breaking strength of the drug containing layer and a breaking strength of the base layer.

7. The microneedle according to claim 6, wherein the breaking strength of the intermediate layer is 0.9 times or less the breaking strength of the drug containing layer and the breaking strength of the base layer.

8. The microneedle according to any one of claims 1 to 7, wherein a boundary between the intermediate layer and the base layer has a flat shape or a shape protruding toward the intermediate layer.

9. The microneedle according to any one of claims 1 to 7, wherein the boundary between the intermediate layer and the base layer has a shape protruding toward the base layer.

10. The microneedle according to any one of claims 1 to 9, wherein shapes of each of the boundaries between the drug containing layer, the intermediate layer, and the base layer are controlled by viscosity and surface activity level of a microneedle forming material, and/or water repellency of a surface of a concave original mold.

11. The microneedle according to any one of claims 1 to 10, wherein a tip side of the drug containing layer further includes a tip layer that contains no drug.

12. The microneedle according to claim 11, wherein a boundary between the tip layer and the drug containing layer has a flat shape or a shape protruding toward the tip layer.

13. The microneedle according to claim 11 or 12, wherein a shape of the boundary between the tip layer and the drug containing layer is controlled by viscosity and surface activity level of a microneedle forming material, and/or water repellency of a surface of a concave original mold.

14. The microneedle according to any one of claims 1 to 13, wherein the intermediate layer is dissolved after the drug containing layer is dissolved in a body.

15. The microneedle according to any one of claims 1 to 14, wherein the drug containing layer is coated with a refractory polymer forming material having lower solubility in the body than the drug containing layer.

16. The microneedle according to any one of claims 1 to 15, comprising two or more drug containing layers.

17. The microneedle according to claim 16, wherein adjacent drug containing layers are partitioned by a boundary layer that contains no drug.

18. The microneedle according to any one of claims 1 to 17, wherein an outer surface is coated with a coating material.

19. The microneedle according to any one of claims 1 to 18, further comprising a partition wall made of a refractory polymer forming material around the drug containing layer,
wherein the partition wall prevents crush of the drug containing layer when the microneedle is implanted, ensures that the drug containing layer is placed in a target area in a focused manner, and releases a drug with a predetermined time delay.

20. The microneedle according to any one of claims 1 to 19, wherein a dissolving time of the drug containing layer and a dissolving time of the partition wall made of the refractory polymer forming material are controlled to control a drug release start time and a drug release time to achieve a programed continuous slow release of the drug.

21. The microneedle according to any one of claims 1 to 20, which is used for targeting administration of the drug.

22. A microneedle patch comprising a plurality of the microneedles according to any one of claims 1 to 21 provided on a substrate.
